# EUROPEAN PATENT SPECIFICATION

(11) **EP 0 768 903 B1**
(45) Date of publication and mention of the grant of the patent: **03.04.2002**
(21) Application number: 95921045.1
(22) Date of filing: 05.06.1995
(51) Int. Cl.: A61M 16/04, A61M 16/00

(54) **A FIBREOPTIC INTUBATING LARYNGEAL MASK AIRWAY**
INTUBATIONSVORRICHTUNG MIT GLASFASEROPTIK UND KEHLKOPFMASKE
DISPOSITIF D'INTUBATION A FIBRES OPTIQUES COMPRENANT UN TUBE TRACHEAL ET UN MASQUE LARYNGE

(30) Priority: 04.06.1994 GB 9411215
(43) Date of publication of application: 23.04.1997
(73) Proprietor: Brain, Archibald Ian Jeremy, Chertsey, Surrey KT16 0DJ (GB)
(72) Inventor: Brain, Archibald Ian Jeremy, Chertsey, Surrey KT16 0DJ (GB)
(74) Representative: West, Alan Harry
(86) International application number: GB9501292
(87) International publication number: WO9533506

(56) References cited:
- WO-A-91/07201
- WO-A-92/13587
- US-A- 4 976 261
- US-A- 5 285 778

## Description

This invention relates to a fibreoptic intubating laryngeal mask airway device for use in anaesthesia.

Laryngeal mask airway devices are described in British Patents 2,111,394 and 2,205,499 and in a number of corresponding foreign patents and patent applications.

Despite the success of such laryngeal mask airway (LMA) devices which are now used in some 40% of all anaesthetic procedures in the United Kingdom, intubation of the trachea remains the ultimate objective of airway management in an emergency or when there may be a risk of inhalation of gastric contents, since the presence of a cuffed tube in the trachea prevents gastric acid present in vomit from entering and damaging the lungs. However, intubation of the trachea is not always possible and, when difficulty is experienced, soiling of the lungs with gastric acid may occur while attempts are being made to intubate. The LMA as described in the above patents has been modified as described in British Patent 2,252,502 in order to facilitate intubation of the trachea using the LMA as a guide, in cases where intubation by conventional means using a laryngoscope to visualise the glottis has failed. However, this intubating laryngeal mask (ILM) has the limitation that, for a high degree of success in passing an endotracheal tube through the ILM tube into the trachea, fibrescopic aid is needed to ensure the endotracheal tube does not pass into the oesophagus or collide with the epiglottis. These hazards, particularly the former which may result in death if undetected, are present also in classical intubation using a laryngoscope. Fibreoptic assisted intubation is another possible technique when classical intubation fails but has the disadvantage that it requires much skill and takes time, a significant drawback in a situation where brain damage or death from lack of oxygen are never more than four minutes away if ventilation cannot be achieved. However, the LMA and the ILM have the advantages that if intubation turns out to be impossible, then the patient can still be kept alive because, unlike the laryngoscope or the fibrescope, the mask part of the LMA or ILM provides an adequate seal around the glottis to permit gentle positive pressure ventilation to be maintained while intubation attempts are taking place. This is an important advantage because in practice death or brain damage occur more often from failure to ventilate the lungs than from lung contamination with gastric contents.

In fibreoptic assisted intubation, the operator has to reach the laryngeal aperture by passing the fibrescope around the back of the tongue (or through the nasal cavity and nasopharynx) and then passing the tip of the scope downwards until the larynx comes into view. This takes time, as previously stated, and because the scope is of small cross-section relative to the cross-section of the pharynx, it is possible for the tip of the fibrescope to wander to one side or the other of the pharynx on the way down, missing the structures of the laryngeal orifice. In addition, the tip of the scope is not protected from contamination with secretions present in the pharynx or from bleeding provoked by its passage, either or both of which may obscure the operator's view. Another problem is that the view is two-dimensional and the field of vision very restricted. The combination of all these factors makes fibreoptic assisted intubation a difficult skill to acquire and maintain. Lastly, fibreoptic scopes are very expensive and not all hospitals are able to afford or maintain them, which adds to the difficulty of ensuring skill is acquired by physicians who might need to use the technique.

These problems are partly resolved when the LMA or ILM is used as a guide for the fibrescope, since when correctly inserted, the mask part of the LMA or ILM completely fills the space of the lower pharynx when the cuff surrounding the mask is inflated. Time to first ventilation is very rapid as the device is passed blindly in a single movement. Thus, when using the LMA, a view of the laryngeal inlet is automatically achieved in the great majority of cases simply by inserting the fibrescope down the tube. In other words, the tube-mask assembly acts as a guide, directing the fibrescope to its target. Furthermore, the inspection can be carried out in a leisurely fashion, since ventilation has already been assured as soon as the LMA cuff is inflated. With the ILM, the probability of viewing the larynx is even greater because unlike the LMA its tube is rigid and provided with an external handle, which permits direct manipulation of the mask relative to the larynx, allowing the clinician to alter the position of the mask if perfect alignment is not achieved in the first instance. However, a fibrescope still has to be inserted in the tube to ascertain whether accurate alignment has been achieved.

A similar problem exists in the device described in WO 91/07201 which is a rigid orolaryngeal/oroesophageal guiding and aiming device for emergency intubation. The device consists of an anatomically contoured guide element which is inserted into the patient's throat to form an upward continuation of the laryngeal wall and has a channel through which an orotracheal tube can be guided into the patient's trachea. Tunnels may be provided through the guide element for blindly guiding other members (for example, a fibreoptic bundle) selectively into the oesophagus or larynx.

The present invention seeks to avoid this problem by incorporating into a modified laryngeal mast airway device one or more fibreoptic systems arranged to provide an optimal and preferably binocular view of the laryngeal inlet. This permits the operator to have immediate optical confirmation of the position of the mask aperture relative to the laryngeal inlet from the moment of insertion of the device and at any time thereafter. A binocular view has the advantage of permitting stereoscopic vision of the anatomy.

In accordance with the present invention, there is now provided a laryngeal mask airway device to facilitate lung ventilation in an unconscious patient, comprising an airway tube and a mask attached to an end of the airway tube, the mask having an annular peripheral formation of roughly elliptical shape and being capable of conforming to and readily fitting within the actual and potential space behind the larynx so as to enable the annular peripheral formation to form a seal around the circumference of the laryngeal inlet without the device penetrating into the interior of the larynx, the annular peripheral formation surrounding the mask into which the airway tube opens, the airway tube being curved and pliable to follow the airway of the patient, the device being characterised by further comprising at least one channel in alignment with the airway tube to accommodate a fibreoptic system for receiving and emitting light directed into the mask, and at least one channel located along the outer radius of the airway tube and in alignment with the airway tube, containing a removable rigid handle by means of which the device may be properly positioned within the patient.

Several advantages accrue from achieving immediate optical confirmation of the position of the mask, as follows.
(a) If regurgitant fluid finds its way into the bowl of the mask before intubation of the trachea can be carried out, then it can immediately be seen and aspirated using a suction catheter before significant lung contamination occurs.
(b) Visual information from the fibrescope can be transferred to a television screen for remote viewing, for example as part of the monitoring equipment on the anaesthetic machine.
(c) As with other monitoring aids, this information can be stored for future use in teaching or as part of the patient's case notes, for example for medico-legal evidence.
(d) The laryngeal view can also be valuable as a teaching aid during routine anaesthesia.
(e) Laryngeal movements indicating inadequate levels of anaesthesia can be seen, permitting early preventive action to avoid the danger of laryngeal spasm or awareness.
(f) The device may be used for diagnosis and treatment of laryngeal or tracheal pathology, for example by ear, nose and throat specialists.
(g) Like the standard LMA, the device can be inserted in the awake patient after application of local anaesthesia to the throat, offering the possibility of treatment and diagnosis of upper airway problems on an outpatient basis.
(h) Most importantly, in the case that intubation of the trachea with an endotracheal tube is desired, the laryngeal view from the mask aperture will help the clinician to guide the tip of the tube towards the laryngeal aperture by manipulating the handle of the rigid laryngeal mask tube through which the endotracheal tube is passed.

Two forms of laryngeal mask airway device in accordance with the invention will now be described by way of example only with reference to the accompanying drawings, in which:
Figure 1 is a side view of a first form of the device; and
Figure 2 is a front view of a second form of the device.

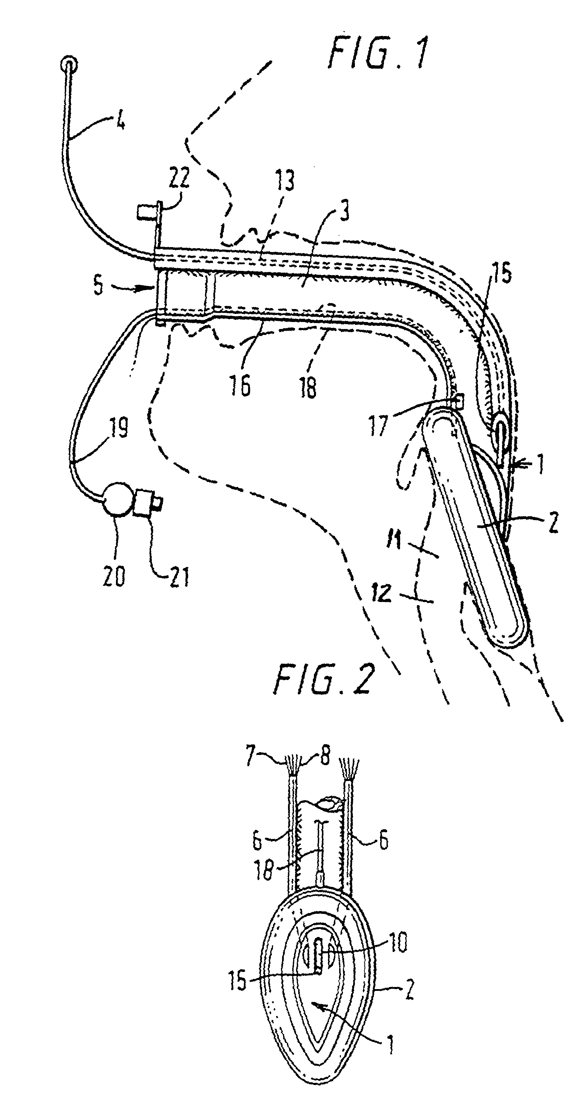

Referring first to Figure 1 of the drawings, a first form of laryngeal mask airway device of the invention comprises a mask body 1 attached to a curved airway tube (3) and surrounded by a generally elliptical peripheral formation, or cuff, 2. The body 1 and cuff 2 are of generally conventional construction and configuration and are described in detail in the patents referred to above. The airway tube is of soft and pliable construction and is made of a plastics material. It has an exterior handle 4 and incorporates a main airway lumen 5 as well as a channel 13 which runs substantially parallel to the airway tube and along its outer radius when the airway tube is in position in the throat of a patient, as shown in the drawing. The rigid handle 4 extends into and along that channel 13 at least during insertion of the device into the patient's throat, and after the device has been properly positioned the handle 4 may be removed.

The channel 13 is open at the outer or mouth end of the device for attachment to plastics disposable suction catheters (not shown) and opens into the mask body 1 where it forms a drain 15 for collection and aspiration of secretions from the lungs, pharynx or stomach, blood, foreign bodies or food particles. The drain 15 and channel 13 are of sufficient diameter to permit passage of such catheters (for example, of 5 to 6 mm diameter in adults).

Optionally, there may further be provided in the wall of main airway tube 3 a small channel 16 (for example, of approximately 1 mm internal diameter) which connects externally of the patient to a tube of similar diameter leading to a device (not shown) known as a capnometer for measuring end-tidal carbon dioxide concentrations in exhaled breath. The channel 16 terminates at gas sampling site 17 within the interior lumen of main airway tube 3 near to its junction with mask 1.

Again optionally, there may be provided a further small channel 18 (for example, of approximately 0.5 mm internal diameter) running longitudinally in the wall of main airway tube 3, the external opening of that channel 18 connecting to a flexible tube 19 which in turn connects with a pilot balloon 20 and self-sealing valve 21 suitable for gas-tight insertion of a disposable syringe for inflation of the device with air. The internal end of that channel 18 communicates with the interior of the inflatable cuff 2 so that the cuff may be inflated or deflated via that channel 18.

Additionally it should be understood that airway tube 3 is provided with easily removable friction-fit (15 mm) connector (not shown) designed for attachment to conventional anaesthetic gas hosing, in order that the device may be used alone to ventilate the lungs of a patient, without using the intubation facility. It should also be understood that to prevent loss of airway pressure, channels 6, 13 and 16 are provided with means (not shown) to prevent leakage of gas through them to the exterior when gas is delivered under pressure through main airway tube 3.

The channel 13 may be of sufficiently large diameter to pass a removable fibreoptic scope. In this connection, the channel will of course present a correspondingly sized drain 15 through which the fibreoptic scope will be able to obtain an acceptable image of the patient's larynx.

In an alternative embodiment of the device of the invention as shown in front view of Figure 2 of the drawings, the device includes one or two additional channels 6 running on one or both sides and substantially parallel to the plane of curvature of the airway tube. The channel(s) 6 accommodate fibreoptic bundles 7 incorporating fibres connected to a light source (not shown) which may be remote from the airway device or incorporated for example in its handle 4. Running alongside the fibreoptic bundles 7 are separate fibre bundles 8 whose functions is to transmit the image of the object so illuminated to the observer's eyes, those separate fibre bundles 8 being sheathed in visible-light impermeable material and running from independently focusable eyepiece or eyepieces 9 situated so as to avoid encumbering intubation attempts. Both fibre bundles 7 and 8 terminate within or close to the airway opening 10 of the mask at such an angle as to offer a view of the larynx and in the case of two separate channels 6 each such channel is suitably directed with respect to the other so as to provide a stereoscopic view of the larynx 11 to the observer viewing through eyepieces 9. Additionally, the combined light-source carrying and viewing fibreoptic bundles 7 and 8 may be moveable within channels 6 so as to permit an observer to obtain varying views of anatomical structures in the region of the larynx. Additionally, again, fibreoptic bundles 7 and 8 may be of sufficient length to permit their entry into the trachea 12 or bronchi should examination of these structures be desired. In the preferred case where there are provided two separate fibreoptic channels 6, fibreoptic bundles 7 and 8 are preferably independently moveable in their respective channels so that it is possible to view simultaneously two different anatomical regions, permitting for example the effect of stimulation or treatment on one region to be seen on another region remote from the region of such treatment or stimulation.

The novel features of the present invention may be incorporated into any form of laryngeal mask device and should not be considered to be restricted to incorporation in the forms of the device detailed above. For example, the gastro-laryngeal mask described in US Patent 5,241, 956 and containing a facility for drainage of oesophageal discharge in addition to an extra posteriorly placed cuff for greater sealing efficacy, may with advantage be adapted to include the features described herein, as may the laryngeal mask fitted with a reflectance type oximeter described in US Patent 5,282,464.

## Claims

1. A laryngeal mask airway device to facilitate lung ventilation in an unconscious patient, comprising an airway tube (3) and a mask (1) attached to an end of the airway tube, the mask having an annular peripheral formation (2) of roughly elliptical shape and being capable of conforming to and readily fitting within the actual and potential space behind the larynx so as to enable the annular peripheral formation to form a seal around the circumference of the laryngeal inlet without the device penetrating into the interior of the larynx (11), the annular peripheral formation surrounding the mask into which the airway tube opens, the airway tube being curved and pliable to follow the airway of the patient, the device being **characterised by** further comprising at least one channel (6,13) in alignment with the airway tube to accommodate a fibreoptic system (7,8) for receiving and emitting light directed into the mask, and at least one channel (13) located along the outer radius of the airway tube and in alignment with the airway tube, containing a removable rigid handle by means of which the device may be properly positioned within the patient.

2. A device according to claim 1, wherein two channels (6) are located along the sides of the airway tube (3) to accommodate the fibreoptic system.

3. A device according to claim 1 or claim 2, wherein the channel (13) aligned with the airway tube (3) opens into the mask (1) forming a drain for secretions from the lungs, pharynx or stomach.

4. A device according to any one of claims 1 to 3, which includes also a still further channel (16) aligned with the airway tube (3) for connection to a capnometer.

## Patentansprüche

1. Kehlkopfmasken-Luftwegvorrichtung zur Erleichterung der Lungenventilation bei einem bewußtlosen Patient, mit einem Luftwegrohr (3) und einer an dessen einem Ende angebrachten Maske (1), die eine ringförmige Randformation (2) von etwa elliptischer Gestalt aufweist und sich anpassen und leicht in den tatsächlichen und potentiellen Raum hinter dem Kehlkopf derart einsetzen läßt, daß die ringförmige Randformation am Umfang des Kehlkopfeingangs eine Dichtung bildet, ohne daß die Vorrichtung in das Innere des Kehlkopfes (11) eindringt, wobei die ringförmige Randformation die Maske, in die das Luftwegrohr mündet, umgibt, und wobei das Luftwegrohr gekrümmt und biegsam ist, so daß es dem Luftweg des Patienten folgen kann, **dadurch gekennzeichnet, daß** die Vorrichtung ferner mindestens einen mit dem Luftwegrohr fluchtenden Kanal (6, 13) zur Aufnahme eines Faseroptiksystems (7, 8) zum Empfangen und Aussenden von in die Maske gerichtetem Licht sowie mindestens einen längs dem Außenradius des Luftwegrohrs angeordneten und mit diesem fluchtenden Kanal (13) mit einem abnehmbaren starren Griff aufweist, mit dem sich die Vorrichtung innerhalb des Patienten ordnungsgemäß positionieren läßt.

2. Vorrichtung nach Anspruch 1, wobei längs den Seiten des Luftwegrohrs (3) zwei Kanäle (6) zur Aufnahme des Faseroptiksystems angeordnet sind.

3. Vorrichtung nach Anspruch 1 oder 2, wobei der mit dem Luftwegrohr (3) fluchtende Kanal (13) in die Maske (1) mündet und eine Drainage für Sekrete aus der Lunge, dem Rachen oder dem Magen bildet.

4. Gerät nach einem der Ansprüche 1 bis. 3 mit einem mit dem Luftwegrohr (3) fluchtenden weiteren Kanal (16) zum Anschluß an ein Kapnometer.

## Revendications

1. Dispositif de masque laryngique de passage d'air pour faciliter la ventilation des poumons d'un patient inconscient, comprenant un tube de passage d'air (3) et un masque (1) attaché à une extrémité du tube de passage d'air, le masque ayant un corps profilé périphérique annulaire (2) de forme globablement elliptique et étant capable de se conformer et de s'adapter facilement à l'espace réel et potentiel derrière le larynx de façon à permettre au corps profilé périphérique annulaire de former un joint autour de la circonférence de l'ouverture du larynx sans que le dispositif ne pénètre à l'intérieur du larynx (11), le corps profilé périphérique annulaire entourant le masque dans lequel le tube de passage d'air s'ouvre, le tube de passage d'air étant incurvé et pliable pour suivre le passage d'air du patient, le dispositif étant **caractérisé en ce qu'**il comprend en outre au moins un canal (6, 13) aligné avec le tube de passage d'air pour loger un système à fibres optiques (7, 8) destiné à recevoir et à émettre de la lumière dirigée dans le masque, et au moins un canal (13) situé le long du rayon extérieur du tube de passage d'air et en alignement avec le tube de passage d'air et contenant un manche ou une poignée rigide amovible au moyen duquel le dispositif peut être placé de façon appropriée à l'intérieur du patient.

2. Un dispositif selon la revendication 1, dans lequel deux canaux (6) sont situés le long des côtés du tube de passage d'air (3) pour loger le système de fibres optiques.

3. Un dispositif selon la revendication 1 ou 2, dans lequel le canal (13) aligné avec le tube de passage d'air (3) s'ouvre dans le masque (1), en formant un drain pour les sécrétions provenant des poumons, du pharynx ou de l'estomac.

4. Un dispositif selon l'une quelconque des revendications 1 à 3, qui comprend également un canal supplémentaire (16) aligné avec le tube de passage d'air (3) pour se connecter à un capnomètre.
